# EUROPEAN PATENT SPECIFICATION

(11) **EP 4 374 810 B1**
(45) Date of publication and mention of the grant of the patent: **28.10.2026**
(21) Application number: 23209303.9
(22) Date of filing: 13.11.2023
(51) Int. Cl.: A61B 18/14, A61B 90/00, A61B 34/10

(54) **GUIDANCE FOR POSITIONING AN ABLATION CATHETER**
FÜHRUNG ZUR POSITIONIERUNG EINES ABLATIONSKATHETERS
GUIDAGE POUR POSITIONNER UN CATHÉTER D'ABLATION

(30) Priority: 14.11.2022 US 202217986854
(43) Date of publication of application: 29.05.2024
(73) Proprietor: Biosense Webster (Israel) Ltd., 2066717 Yokneam (IL)
(72) Inventor: ZOUBI, Alaa, 2066717 Yokneam (IL); ROSENBERG, Avigdor, 2066717 Yokneam (IL); MASSARWA, Fady, 2066717 Yokneam (IL)
(74) Representative: Carpmaels & Ransford LLP

(56) References cited:
- EP-A1- 3 791 819
- US-A1- 2005 033 281
- US-A1- 2013 150 693
- US-A1- 2020 022 653
- US-A1- 2020 030 028
- US-A1- 2022 047 326
- US-A1- 2022 192 753

## Description

### FIELD OF THE DISCLOSURE

The present disclosure is related generally to the field of electrophysiology, and particularly to computer-implemented techniques for facilitating an electrophysiological procedure.

### BACKGROUND

In some electrophysiological procedures, a pulmonary vein is electrically isolated from the myocardium by the ablation of tissue at the ostium of the vein.

US 2022/047326 A1 describes a system that includes a catheter, a switching assembly, and a processor. The catheter including an expandable frame, which is coupled to a distal end of the catheter, and multiple electrodes, which are disposed on the expandable frame in a radial geometry. The switching assembly is electrically connected to the catheter, and is configured to electrically short between selected ones of the electrodes. The processor is configured, for first and second disjoint groups of the electrodes, to control the switching assembly to electrically short the electrodes within each of the first and second groups, for applying one or more bipolar ablation energy between the first and second groups when the electrodes are placed in contact with a target tissue of the organ.

US 2020/030028 A1 describes an apparatus and methods for determining positioning of a energy delivery element including deploying a energy delivery element at a treatment site proximal to a vessel wall; using a multi-region pressure sensing apparatus to sense pressures applied in a plurality of directions about the energy delivery element; and determining an orientation of the energy delivery element based on the pressures measured in the plurality of directions about the energy delivery element.

### SUMMARY 1

The invention is defined by claim 1. Further embodiments of the invention are defined by the dependent claims. 1

### BRIEF DESCRIPTION OF THE DRAWINGS

The present disclosure will be more fully understood from the following detailed description of examples thereof, taken together with the drawings, in which:
Fig. 1 is a schematic illustration of an ablation system, in accordance with some examples of the present disclosure;
Figs. 2A-B are schematic illustrations of an iterative process for facilitating an ablation, in accordance with some examples of the present disclosure;
Fig. 3 is a flow diagram for an algorithm for facilitating an ablation, in accordance with some examples of the present disclosure; and
Fig. 4 is a schematic illustration of a rotation computation, in accordance with some examples of the present disclosure.

### DETAILED DESCRIPTION OF EXAMPLES

### OVERVIEW

In the context of the present application, including the claims, the "pose" of an object refers to a combination of the location and orientation of the object with respect to a referential datum, such that a first pose may differ from a second pose by a translation and/or a rotation.

In some cases, an intrabody ablation catheter is used to ablate the ostium of a pulmonary vein. Such a catheter typically comprises a distal structure, such as a balloon, having a plurality of electrodes disposed thereon. The electrodes are typically arranged (e.g., evenly distributed) around the circumference of the structure.

In such cases, it is typically desirable to contact the ostium with as many of the electrodes as possible, so as to ablate around the full circumference of the ostium. However, even with continual updates as to the number of electrodes contacting the ostium, it may be difficult for the physician to determine the required adjustments to the pose of the structure.

To address this challenge, examples of the present disclosure provide a processor configured to compute, repeatedly, a relatively small pose adjustment, such as a translation or rotation, that is likely to increase the number of contacting electrodes. After each computation, the processor displays two icons, each of which represents the structure and is typically rendered in three dimensions. One of the icons, a "target-pose icon," is offset from the other icon, a "current-pose icon," per the computed adjustment. The physician may then adjust the pose of the structure, while viewing the display, so as to bring the current-pose icon into alignment with the target-pose icon.

As the present inventors have observed, a smaller number of contacting electrodes generally indicates that the structure has not fully entered the ostium, whereas a greater number of contacting electrodes generally indicates that the structure has fully entered the ostium but is not optimally oriented. Hence, in some examples, the processor compares the number of contacting electrodes to a predefined threshold. If the number is less than the threshold, the processor computes a translation of the distal structure, typically along the proximal-distal axis of the structure. Otherwise, the processor computes a rotation with respect to the proximal-distal axis or with respect to another referential axis defined by the distal structure.

### SYSTEM DESCRIPTION

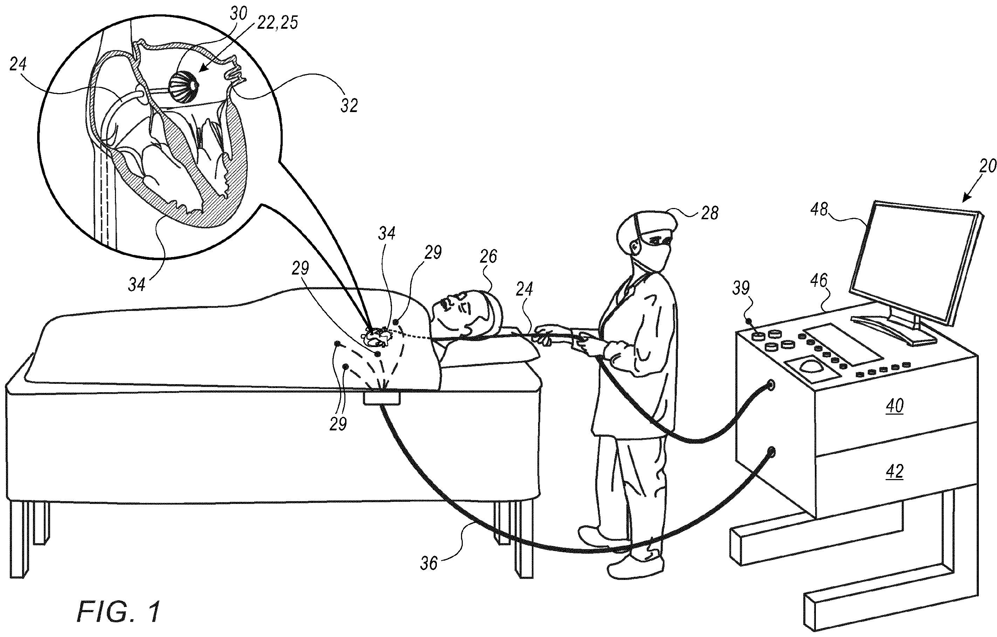

Reference is initially made to Fig. 1, which is a schematic illustration of an ablation system 20, in accordance with some examples of the present disclosure.

System 20 comprises a catheter 24 configured for insertion into the body of a subject 26 by a physician 28. System 20 further comprises a console 46 comprising controls 39 for controlling system 20, and a display 48, which is typically mounted on console 46.

System 20 further comprises a processor 40 and a signal generator 42, which are typically contained within console 46. System 20 may further comprise a volatile memory, a non-volatile memory, a noise filter, an analog-to-digital (A/D) converter, and/or any other suitable circuitry.

The distal end of catheter 24 comprises a distal structure 22 comprising multiple electrodes 30, each of which is connected to signal generator 42 via a wire passing through catheter 24. Typically, distal structure 22 comprises an expandable member 25, such as a balloon (as shown in Fig. 1), an expandable basket of spines, or an expandable planar structure. Electrodes 30 are typically distributed around the circumference or perimeter of distal structure 22.

Subsequently to inserting catheter 24 into the body of subject 26, physician 28 navigates distal structure 22 of catheter 24 to the left atrium of the heart 34 of the subject. Subsequently, with the help of processor 40 (as described below with reference to the subsequent figures), the physician positions distal structure 22 within the ostium of a pulmonary vein 32. Subsequently, using controls 39, the physician causes signal generator 42 to generate ablating signals, such that the ablating signals pass through the tissue of the ostium via electrodes 30.

Processor 40 is configured to track the pose of distal structure 22.

For example, distal structure 22 may comprise one or more electromagnetic sensors connected to console 46, and system 20 may further comprise one or more magnetic-field generators configured to generate a magnetic field as a referential datum. In particular, the magnetic field may induce tracking signals in the electromagnetic sensors so that the positions of the sensors can be triangulated, and hence determined with respect to the referential datum. In other words, based on the tracking signals, processor 40 may ascertain the respective locations of the sensors with respect to the referential datum, and hence, the pose of the distal structure. Such location-tracking techniques are well known and previously disclosed, for example, in US Patents 5,391,199, 5,443,489, and 6,788,967 to Ben-Haim, in US Patent 6,690,963 to Ben-Haim et al., in US Patent 5,558,091 to Acker et al., and in US Patent 6,177,792 to Govari.

Alternatively or additionally, system 20 may further comprise one or more reference electrodes 29 coupled to the surface of the body of the subject and connected to console 46 via a cable 36. Electric currents at predefined frequencies for electrodes 30 may be passed between electrodes 30 (and/or other electrodes on the distal structure) and reference electrodes 29. Based on the resulting impedances measured by reference electrodes 29, processor 40 may ascertain the respective locations of electrodes 30 and hence, the pose of the distal structure, by triangulation of the impedance values with reference electrodes 29. Such techniques may utilize a location map calibrated, in advance, using electromagnetic sensors, as described, for example, in US Patent 7,536,218 to Govari et al. and US Patent 8,456,182 to Bar-Tal et al.
Alternatively or additionally, electric currents may be passed between reference electrodes 29. Based on the resulting voltages at electrodes 30 (and/or other electrodes on the distal structure), processor 40 may ascertain the respective locations of electrodes 30, and hence, the pose of the distal structure. Such techniques are described, for example, in US Patent 5,983,126 to Wittkampf and US Patent 5,944,022 to Nardella.

In some examples, system 20 further comprises one or more electrocardiogram (ECG) electrodes, which are electrically coupled to the skin of subject 26. Processor 40 may receive the ECG signals acquired by the ECG electrodes via a cable connected to console 46.

While distal structure 22 is within heart 34, the processor receives signals indicating the degree to which each electrode 30 is in contact with the tissue. For example, the signals may indicate the impedance seen by each electrode. (The impedance of tissue is greater than that of blood, such that a higher impedance indicates greater contact.) Alternatively or additionally, the signals may indicate the temperature measured by thermocouples co-located with the electrodes. (Typically, a lower temperature indicates greater contact, due to less blood flow near the thermocouples and hence, less heating of the thermocouples by blood.) Alternatively or additionally, the signals may indicate a force or pressure measured by sensors co-located with the electrodes.

In general, processor 40 may be embodied as a single processor, or as a cooperatively networked or clustered set of processors. The functionality of processor 40 may be implemented solely in hardware, e.g., using one or more fixed-function or general-purpose integrated circuits, Application-Specific Integrated Circuits (ASICs), and/or Field-Programmable Gate Arrays (FPGAs). Alternatively, this functionality may be implemented at least partly in software. For example, processor 40 may be embodied as a programmed processor comprising, for example, a central processing unit (CPU) and/or a Graphics Processing Unit (GPU). Program code, including software programs, and/or data may be loaded for execution and processing by the CPU and/or GPU. The program code and/or data may be downloaded to the processor in electronic form, over a network, for example. Alternatively or additionally, the program code and/or data may be provided and/or stored on non-transitory tangible media, such as magnetic, optical, or electronic memory. Such program code and/or data, when provided to the processor, produce a machine or special-purpose computer, configured to perform the tasks described herein.

### FACILITATING AN ABLATION

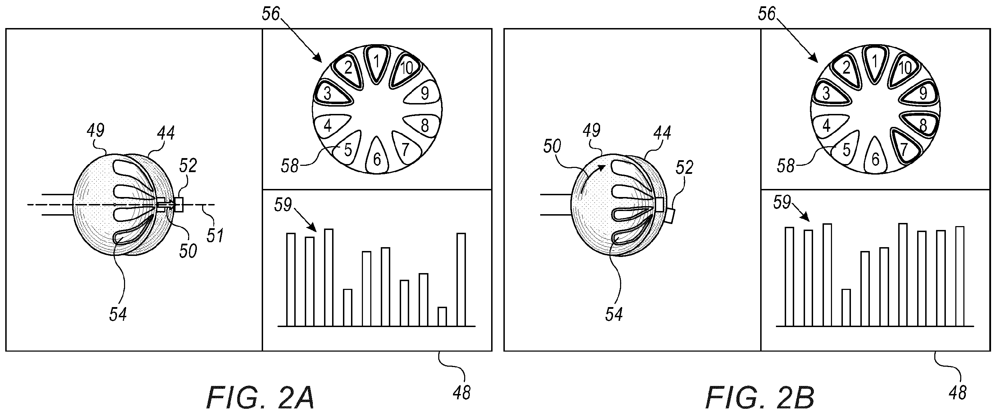

Reference is now made to Figs. 2A-B, which are schematic illustrations of an iterative process for facilitating an ablation, in accordance with some examples of the present disclosure.

In general, for an ablation of a pulmonary vein, it is best to maximize the number of electrodes 30 (Fig. 1) that contact the ostium of the vein. To facilitate this maximizing, processor 40 (Fig. 1) continually ascertains the number of electrodes contacting tissue. For example, for each electrode, the processor may receive an impedance-indicating, force-indicating, or temperature-indicating signal. If the indicated impedance or force exceeds a threshold, or if the indicated temperature is below a threshold, the electrode may be deemed to be contacting tissue.

Prior to the procedure, a threshold number of electrodes is defined. This predefined threshold, referred to herein as a "lower threshold," is the minimum number of electrodes that need to contact the tissue in order to assume that distal structure 22 is positioned at least partly within the ostium. Typically, the lower threshold is at least 20% of the total number of electrodes around the circumference of the distal structure.

In response to the number of contacting electrodes equaling or exceeding the lower threshold, the processor carries out an iterative process for increasing the number of electrodes that contact the ostium. During each iteration of the process, the processor compares the number of contacting electrodes to another predefined threshold, referred to herein as a "higher threshold." In response to the number being less than the higher threshold, the processor computes an adjustment to the pose of the distal structure. The processor then displays, on display 48, a target-pose icon 44, which represents the distal structure, offset, per the adjustment, from a current-pose icon 49 representing the distal structure at the current pose of the distal structure. (Subsequently to the initial display of current-pose icon 49 and target-pose icon 44, the current-pose icon may move - and hence, the offset may change - cyclically, due to the effect of respiratory motion on the pose of the distal structure.)

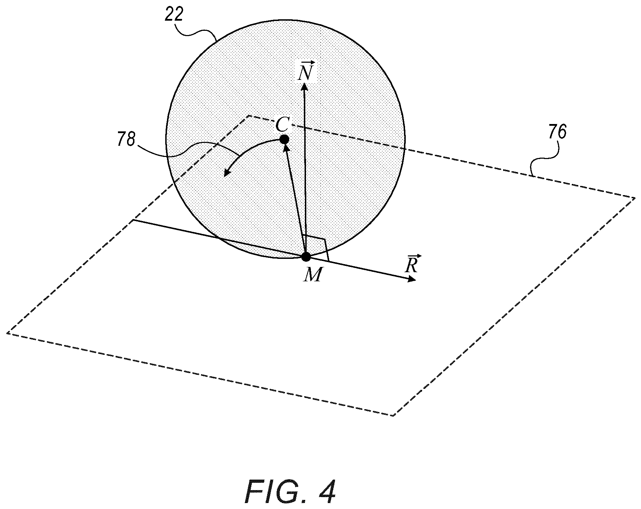

For example, as shown in Fig. 2A, the processor may compute a translation, e.g., along the proximal-distal (or "longitudinal") axis 51 of the distal structure. (It is noted that proximal-distal axis 51 is typically not displayed.) Alternatively, as shown in Fig. 2B, the processor may compute a rotation, e.g., as further described below with reference to Fig. 4. Alternatively, the processor may compute both a rotation and a translation.

Typically, the two icons are rendered in three dimensions, so as to increase the clarity of the required pose adjustment. Target-pose icon 44 is typically displayed "behind" current-pose icon 49 as in Figs. 2A and 2B. In some embodiments, the two icons are shaded or colored differently from one another, so as to facilitate differentiating between the two.

Typically, current-pose icon 49 includes multiple electrode icons 54, which represent different respective electrodes 30 (Fig. 1). In some examples, the processor modifies (e.g., highlights or changes the color of) those electrode icons 54 representing electrodes in contact with the tissue.

Current-pose icon 49 may be rendered from any suitable perspective. In some examples, the perspective may be adjusted by the physician (or by any other user).

In response to viewing target-pose icon 44, the physician begins adjusting the pose of the distal structure while continuing to look at the display. During the adjustment, the processor continually refreshes current-pose icon 49 so as to indicate the current pose of the distal structure. Upon the distal structure reaching the target pose, the current-pose icon overlaps the target-pose icon.

Subsequently, the next iteration of the iterative process begins, and target-pose icon 44 is refreshed. In this manner, the pose of the distal structure may be incrementally adjusted until the number of contacting electrodes equals or exceeds the higher threshold.

In some examples, the processor displays an adjustment indicator 50 indicating the computed adjustment. For example, as shown in Fig. 2A, for a translation, the processor may display an arrow pointing in the direction of the translation. For a translation along proximal-distal axis 51, the arrow may begin at the base of the distal nose 52 of current-pose icon 49, and terminate at the base of distal nose 52 of target-pose icon 44.

Typically, the processor displays a schematic rendering 56 of a cross-section through the distal structure at the electrodes. In rendering 56, the electrodes are represented by different respective cross-sectional electrode icons 58, which may be numbered (e.g., as 1, 2, 3, 4, 5, 6, 7, 8, 9, 10) so as to indicate the electrode to which each cross-sectional electrode icon 58 corresponds. In some examples, the processor modifies (e.g., highlights or changes the color of) those cross-sectional electrode icons 58 representing electrodes in contact with the tissue.

Alternatively or additionally, the processor may display a graph 59 indicating a measure of contact (e.g., an impedance, force, or temperature) for each of the electrodes.

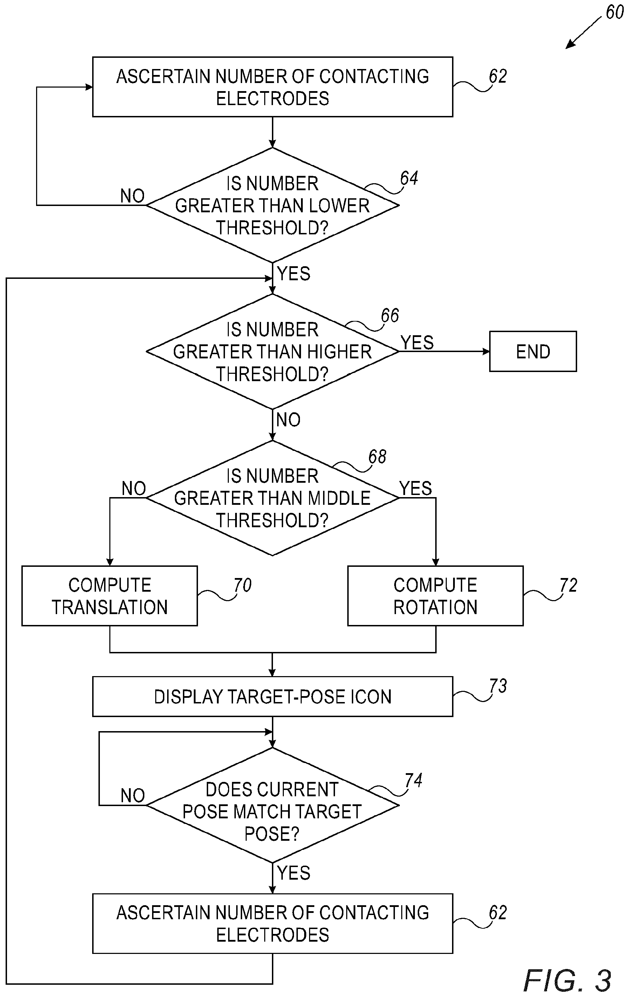

Reference is now made to Fig. 3, which is a flow diagram for an algorithm 60 for facilitating an ablation, in accordance with some examples of the present disclosure. Algorithm 60 may be executed by processor 40 (Fig. 1).

Algorithm 60 begins with an ascertaining step 62, at which the processor ascertains the numbers of electrodes contacting the tissue of the subject. Subsequently to ascertaining the number, the processor checks, at a checking step 64, whether the number is greater than the lower threshold. If not, the processor returns to ascertaining step 62, typically after a predefined delay. Otherwise, the processor begins execution of an iterative process.

The iterative process begins at another checking step 66, at which the processor checks whether the number of contacting electrodes is greater than the higher threshold. If yes, execution of the algorithm ends. Otherwise, the processor computes and displays an adjustment to the pose of the distal structure, as described above with reference to Figs. 2A-B.

Typically, the type of adjustment that is computed depends on the number of contacting electrodes. For example, if the number is less than a middle threshold, which is between the lower threshold and the higher threshold and in some examples is at least 50% of the total number of the electrodes around the circumference of the distal structure, the processor may compute a translation, as shown in Fig. 2A, at a translation-computing step 70. Otherwise, the processor may compute a rotation, as shown in Fig. 2B, at a rotation-computing step 72.

Typically, the pose adjustment computed by the processor is relatively small. For example, the translation computed at translation-computing step 70 may be less than 5 mm, and/or the rotation computed at rotation-computing step 72 may be less than 10 degrees. Thus, advantageously, the pose of the distal structure may be adjusted incrementally.

Subsequently to computing the pose adjustment, the processor displays the target-pose icon, at an icon-displaying step 73, so as to indicate the adjustment. Next, as the physician adjusts the pose of the distal structure, the processor continually checks, at another checking step 74, whether the current pose matches, i.e., is within a predefined threshold of, the target pose. Typically, while the continual checking is performed, the processor continually refreshes current-pose icon 49 (Figs. 2A-B) so as to indicate any changes to the pose of the distal structure.

In response to the current pose matching the target pose, the processor again performs ascertaining step 62, and then returns to checking step 66 for the next iteration of the process.

Reference is now made to Fig. 4, which is a schematic illustration of a rotation computation, in accordance with some examples of the present disclosure.

In some examples, to compute a rotation, the processor first computes a centroid M of those of the electrodes contacting the ostium and a centroid C of distal structure 22 of the catheter, e.g., of expandable member 25 (Fig. 1). In computing these centroids, the processor may assign a uniform weight to distal structure 22, including electrodes 30. Alternatively, for at least one of the centroid computations, the processor may assign different weights to different respective portions of the distal structure. For example, in computing M, the processor may assign a respective weight to each of electrodes 30 contacting the ostium as an increasing function (e.g., an increasing linear function) of a degree to which the electrode contacts the ostium. For example, the weight may be an increasing function of the impedance seen by the electrode and/or the force applied to the electrode, and/or a decreasing function of the temperature measured at electrode 30.

Subsequently, the processor computes a normal vector N, which is normal to a hypothetical plane 76 tangent to the distal structure at the centroid M. The processor further computes an axis-of-rotation vector *̅R̅*̅ as *N̅ x* (̅*̅C̅ -̅ M̅*̅)̅*.* Finally, the processor computes the rotation about R, as indicated in Fig. 4 by a rotation indicator 78. Typically, the rotation is toward the distal end of distal structure 22, such as toward distal nose 52 (Fig. 2B).

It will be appreciated by persons skilled in the art that the present disclosure is not limited to what has been particularly shown and described hereinabove. Rather, the scope of examples of the present disclosure includes both combinations and subcombinations of the various features described hereinabove, as well as variations and modifications thereof that are not in the prior art, which would occur to persons skilled in the art upon reading the foregoing description.

The invention is defined by the following claims.

## Claims

1. A system (20) for use with a catheter (24) having a distal structure (22) including a plurality of electrodes (30), the system comprising:
a display (48); and
a processor (40), configured to:
ascertain a number of the plurality of electrodes of the distal structure contacting an ostium of a pulmonary vein (32), **characterised in that**, the processor (40) is further configured to in response to the number equaling or exceeding a predefined lower threshold, carry out an iterative process including, during each iteration of the process:
in response to the number being less than a predefined higher threshold, computing an adjustment to a current pose of the distal structure, and
displaying, on the display, a target-pose icon (44), which represents the distal structure, offset, per the adjustment, from a current-pose icon (49) representing the distal structure at the current pose.

2. The system (20) according to claim 1, wherein the displaying includes rendering the target-pose icon (44) and the current-pose icon (49) in three dimensions.

3. The system (20) according to claim 1, wherein the lower threshold is at least 20% of a total number of the electrodes (30) around a circumference of the distal structure (22).

4. The system (20) according to any preceding claim, wherein computing the adjustment includes:
computing a translation of the distal structure in case the number is less than a predefined middle threshold, which is between the lower threshold and the higher threshold, or
computing a rotation of the distal structure in case the number is equal to or greater than the middle threshold.

5. The system (20) according to claim 4, wherein the translation is along a proximal-distal axis (51) of the distal structure (22).

6. The system (20) according to claim 4, wherein the middle threshold is at least 50% of a total number of the electrodes (30) around a circumference of the distal structure (22).

7. The system (20) according to claim 4, wherein computing the rotation includes:
computing a first centroid M of those of the plurality of electrodes (30) contacting the ostium and a second centroid C of the distal structure (22),
computing a normal vector N , which is normal to a hypothetical plane (76) tangent to the distal structure at the first centroid,
computing an axis-of-rotation vector *̅R̅*̅ as *N̅ x* (̅*̅C̅ -̅ M̅*̅)̅*,* and
computing the rotation about R.

8. The system (20) according to claim 7, wherein computing the first centroid includes assigning a respective weight to each of the electrodes (30) contacting the ostium as an increasing function of a degree to which the electrode contacts the ostium.

9. The system according to claim 4, wherein the translation is less than 5 mm.

10. The system according to claim 4, wherein the rotation is less than 10 degrees.

## Patentansprüche

1. System (20) zur Verwendung mit einem Katheter (24), der eine distale Struktur (22) aufweist, einschließlich einer Vielzahl von Elektroden (30), das System umfassend:
eine Anzeige (48); und
einen Prozessor (40), der konfiguriert ist zum:
Ermitteln einer Anzahl der Vielzahl von Elektroden der distalen Struktur, die ein Ostium einer Pulmonalvene (32) kontaktieren,
**dadurch gekennzeichnet, dass** der Prozessor (40) ferner konfiguriert ist zum,
als Reaktion darauf, dass die Anzahl gleich einem vorgegebenen unteren Schwellenwert ist oder diesen überschreitet, Ausführen eines iterativen Prozesses, der während jeder Iteration des Prozesses einschließt:
als Reaktion darauf, dass die Anzahl kleiner als ein vorgegebener höherer Schwellenwert ist, Berechnen einer Anpassung einer aktuellen Pose der distalen Struktur, und
Anzeigen, auf der Anzeige, eines Symbols (44) einer Zielpose, das die distale Struktur darstellt, das gemäß der Anpassung von einem Symbol (49) der aktuellen Pose versetzt ist, das die distale Struktur an der aktuellen Pose darstellt.

2. System (20) nach Anspruch 1, wobei das Anzeigen ein Rendern des Symbols (44) der Zielpose und des Symbols (49) der aktuellen Pose in drei Dimensionen einschließt.

3. System (20) nach Anspruch 1, wobei der untere Schwellenwert mindestens 20 % einer gesamten Anzahl der Elektroden (30) um einen Umfang der distalen Struktur (22) herum beträgt.

4. System (20) nach einem der vorstehenden Ansprüche, wobei das Berechnen der Anpassung einschließt:
Berechnen einer Translation der distalen Struktur, falls die Anzahl kleiner als ein vorgegebener mittlerer Schwellenwert ist, der zwischen dem unteren Schwellenwert und dem höheren Schwellenwert liegt, oder
Berechnen einer Drehung der distalen Struktur, falls die Anzahl gleich oder größer als der mittlere Schwellenwert ist.

5. System (20) nach Anspruch 4, wobei die Translation entlang einer proximal-distalen Achse (51) der distalen Struktur (22) erfolgt.

6. System (20) nach Anspruch 4, wobei der mittlere Schwellenwert mindestens 50 % einer gesamten Anzahl der Elektroden (30) um einen Umfang der distalen Struktur (22) herum beträgt.

7. System (20) nach Anspruch 4, wobei das Berechnen der Drehung einschließt:
Berechnen eines ersten Schwerpunkts M derjenigen der Vielzahl von Elektroden (30), die das Ostium kontaktieren, und eines zweiten Schwerpunkts C der distalen Struktur (22),
Berechnen eines Normalenvektors *N̅,* der normal zu einer hypothetischen Ebene (76) ist, die tangential zu der distalen Struktur an dem ersten Schwerpunkt ist,
Berechnen eines Rotationsachsenvektors *R̅ als N̅* x (̅*̅C̅*̅-̅*̅M̅*̅)̅, und Berechnen der Rotation um *̅R̅*̅.

8. System (20) nach Anspruch 7, wobei das Berechnen des ersten Schwerpunkts ein Zuweisen eines jeweiligen Gewichts zu jeder der Elektroden (30), die das Ostium kontaktieren, als eine steigende Funktion eines Grades, zu dem die Elektrode das Ostium kontaktiert, einschließt.

9. System nach Anspruch 4, wobei die Translation kleiner als 5 mm ist.

10. System nach Anspruch 4, wobei die Drehung kleiner als 10 Grad ist.

## Revendications

1. Système (20) pour une utilisation avec un cathéter (24) ayant une structure distale (22) comportant une pluralité d'électrodes (30), le système comprenant :
un dispositif d'affichage (48) ; et
un processeur (40), configuré pour :
déterminer un nombre de la pluralité d'électrodes de la structure distale en contact avec un ostium d'une veine pulmonaire (32) **caractérisé en ce que** le processeur (40) est en outre configuré pour
en réponse au fait que le nombre est égal ou supérieur à un seuil inférieur prédéfini, exécuter un processus itératif comportant, pendant chaque itération du processus :
en réponse au fait que le nombre est inférieur à un seuil supérieur prédéfini, le calcul d'un ajustement d'une pose actuelle de la structure distale, et
l'affichage, sur le dispositif d'affichage, d'une icône de pose cible (44), qui représente la structure distale, décalée, selon l'ajustement, par rapport à une icône de pose actuelle (49) représentant la structure distale à la pose actuelle.

2. Système (20) selon la revendication 1, dans lequel l'affichage comporte le rendu de l'icône de pose cible (44) et de l'icône de pose actuelle (49) en trois dimensions.

3. Système (20) selon la revendication 1, dans lequel le seuil inférieur est d'au moins 20 % d'un nombre total des électrodes (30) autour d'une circonférence de la structure distale (22).

4. Système (20) selon l'une quelconque des revendications précédentes, dans lequel le calcul de l'ajustement comporte :
le calcul d'une translation de la structure distale dans le cas où le nombre est inférieur à un seuil intermédiaire prédéfini, qui est compris entre le seuil inférieur et le seuil supérieur, ou
le calcul d'une rotation de la structure distale dans le cas où le nombre est égal ou supérieur au seuil intermédiaire.

5. Système (20) selon la revendication 4, dans lequel la translation s'effectue le long d'un axe proximal-distal (51) de la structure distale (22).

6. Système (20) selon la revendication 4, dans lequel le seuil intermédiaire est d'au moins 50 % d'un nombre total des électrodes (30) autour d'une circonférence de la structure distale (22).

7. Système (20) selon la revendication 4, dans lequel le calcul de la rotation comporte :
le calcul d'un premier centroïde M de celles de la pluralité d'électrodes (30) entrant en contact avec l'ostium et d'un second centroïde C de la structure distale (22),
le calcul d'un vecteur normal *̅N̅*̅, qui est normal à un plan hypothétique (76) tangent à la structure distale au niveau du premier centroïde,
le calcul d'un vecteur d'axe de rotation *R̅ sous la forme N̅ x* (̅*̅C̅ -̅ M̅*̅)̅*,* et le calcul de la rotation autour de *̅R̅*̅.

8. Système (20) selon la revendication 7, dans lequel le calcul du premier centroïde comporte l'attribution d'un poids respectif à chacune des électrodes (30) entrant en contact avec l'ostium en fonction croissante d'un degré auquel l'électrode entre en contact avec l'ostium.

9. Système selon la revendication 4, dans lequel la translation est inférieure à 5 mm.

10. Système selon la revendication 4, dans lequel la rotation est inférieure à 10 degrés.
